# FASCICULE DE BREVET EUROPEEN

(11) **EP 0 478 428 B1**
(45) Date de publication et mention de la délivrance du brevet: **08.03.1995**
(21) Numéro de dépôt: 91402515.0
(22) Date de dépôt: 20.09.1991
(51) Int. Cl.: C01B 35/10, C07C 35/08, C07C 29/52

(54) **Procédé de préparation d'oxyde borique par hydrolyse du borate de méthyle et sa mise en oeuvre dans l'oxydation d'hydrocarbures saturées en alcools**
Verfahren zur Herstellung von Boroxid durch Hydrolyse von Methylborat und seine Anwendung bei der Oxidation von gesättigten Kohlenwasserstoffen zu Alkoholen
Process for the preparation of boron oxide by hydrolysis of methylborate and its use in the oxidation of saturated hydrocarbons to alcohols

(30) Priorité: 25.09.1990 FR 9011910; 08.10.1990 FR 9012473
(43) Date de publication de la demande: 01.04.1992
(73) Titulaire: INSTITUT FRANCAIS DU PETROLE, 92506 Rueil-Malmaison Cédex (FR)
(72) Inventeur: Alagy, Jacques, F-69260 Charbonnieres (FR); Busson, Christian, F-69260 Charbonnieures (FR); Asselineau, Lionel, F-75017 Paris (FR)

(56) Documents cités:
- FR-A- 1 423 382
- FR-A- 2 363 516
- US-A- 2 808 424
- US-A- 2 833 623
- US-A- 3 232 704
- US-A- 3 895 067
- US-A- 4 058 565
- Steinberg H. "Organoboron Chemistry" Vol 1 (1964) Chapitre IV page 142 (Interscience publishers)

## Description

L'invention concerne un procédé de préparation d'oxyde borique sous forme anhydre ou hydratée à partir de borate de méthyle ou d'un mélange de borate de méthyle et d'un tiers corps, par exemple un solvant du borate de méthyle tel que, en particulier, le méthanol.

L'invention concerne également un procédé d'oxydation d'hydrocarbures saturés en un produit comprenant les alcools correspondant à ces hydrocarbures, et plus particulièrement un procédé d'oxydation d'au moins un hydrocarbure saturé choisi dans le groupe formé par le cyclohexane et les hydrocarbures saturés acycliques et cycliques ayant de 7 à 20 atomes de carbone dans leur molécule, ladite oxydation étant effectuée à l'aide d'un gaz contenant de l'oxygène moléculaire en présence d'au moins un composé de bore formant des esters avec l'alcool formé au cours de l'oxydation et ledit procédé comprenant une récupération des composés de bore à partir des esters boriques obtenus et une réutilisation de ces composés à l'étape d'oxydation.

Les composés de bore de formule générale (I) B₂O₃, x H₂O dans laquelle x représente un nombre de 0 à 3 sont des composés décrits dans l'art antérieur et qui sont en particulier utilisés comme co-réactif pour l'oxydation des hydrocarbures saturés en alcools et cétones. Dans ces réactions d'oxydation, le co-réactif à base de bore que l'on utilise le plus fréquemment comprend une majorité de composé de bore de formule générale (I) dans laquelle x est égal à 1 habituellement dénommé acide métaborique. Ce co-réactif, généralement insoluble dans le milieu réactionnel, doit être de préférence sous une forme extrêmement divisée de manière à avoir une activité chimique maximale et le taux d'impuretés organiques présentes doit de préférence être le plus faible possible car elles sont néfastes pour l'oxydation et provoquent souvent un encrassement important des réacteurs.

L'hydrolyse du borate de méthyle en présence ou en absence de méthanol est un procédé connu dans l'art antérieur et en particulier décrit par R. F. NICKERSON dans J. Inorg. Nucl. Chem., 1971, Vol. 33, pages 1665-1671. Cette réaction d'hydrolyse est une réaction équilibrée et complexe fournissant en particulier un mélange pouvant contenir les divers composés de formule générale (I) ci-avant. D'après NICKERSON cité ci-avant, il n'est pas possible de déplacer l'équilibre d'hydrolyse par récupération du ou des composés formés par suite d'une perte très importante de bore durant l'évaporation. Par ailleurs, le déplacement de l'équilibre par simple distillation du méthanol, formé au cours de la réaction d'hydrolyse, n'est pas possible à cause de l'azéotrope à point d'ébullition minimum que forme ce composé avec le borate de méthyle.

Il a été proposé par ailleurs d'effectuer la distillation du produit d'hydrolyse en présence d'un composé formant un hétéroazéotrope avec le méthanol, cet hétéroazéotrope ayant un point d'ébullition inférieur à celui de l'azéotrope borate de méthyle-méthanol.

Cette méthode est décrite par exemple dans l'ouvrage de Howard STEINBERG "Organoboron Chemistry" Vol 1 (1964), Chapitre IV page 42 Interscience Publishers.
Le composé considéré peut être le méthyl-2 pentane ou le diméthyl-2,3 butane.

Le procédé de préparation d'au moins un oxyde borique, sous forme anhydre ou hydratée, de formule générale (I) ci-avant, de la présente invention, permet d'obtenir, avec une très bonne conversion habituellement supérieure à 95 % en moles et le plus souvent d'environ 100 %, un produit extrêmement divisé et relativement pur. Lorsque le produit que l'on forme par hydrolyse du borate de méthyle (de formule B(OCH₃)₃) doit être utilisé comme co-réactif d'oxydation, il est habituellement préférable de choisir les conditions opératoires, notamment la quantité d'eau employée par rapport à la quantité de borate de méthyle mise en jeu, de manière à ce qu'il contienne moins de 70 %, de préférence moins de 50 % et le plus souvent moins de 30 % en poids d'acide orthoborique de formule H₃BO₃ (B₂O₃,3H₂O).

De façon plus précise, la présente invention concerne un procédé de préparation d'au moins un oxyde borique, sous forme anhydre ou hydratée, de formule générale B₂O₃,xH₂O dans laquelle x est un nombre de 0 à 3, par hydrolyse de borate de méthyle, dans lequel on introduit dans une zone d'hydrolyse du borate de méthyle et de l'eau dans un rapport molaire d'au moins 1,3 mole d'eau, de préférence d'au moins 1,5 mole d'eau, par mole de borate de méthyle, puis on introduit dans une colonne à distiller le produit issu de la zone d'hydrolyse, comprenant ledit oxyde borique et du méthanol (formés par hydrolyse du borate de méthyle), et au moins un composé formant un hétéroazéotrope avec le méthanol, ledit hétéroazéotrope ayant un point d'ébullition inférieur à celui de l'azéotrope borate de méthyle-méthanol, caractérisé en ce que :
- on introduit également dans ladite colonne au moins un composé ayant un point d'ébullition supérieur à celui du borate de méthyle, ledit composé ne formant pas d'azéotrope à point d'ébullition inférieur à celui dudit hétéroazéotrope,
- on récupère, en tête de colonne, ledit hétéroazéotrope que l'on envoie dans un décanteur dans lequel on sépare le méthanol du composé formant ledit hétéroazéotrope et on recycle ce composé dans la colonne à distiller à titre d'alimentation de ladite colonne en ce composé et de reflux, et
- on récupère, en fond de colonne, un mélange d'au moins un oxyde borique, sous forme anhydre ou hydratée, et du composé à point d'ébullition supérieur à celui du borate de méthyle.

La réaction d'hydrolyse permet en particulier d'obtenir les oxydes de formule générale (I) dans laquelle x est égal à zéro, un ou trois, souvent respectivement dénommés anhydride borique (B₂O₃), acide métaborique (HBO₂ ou B₂O₃, H₂O) et acide orthoborique (H₃BO₃ ou B₂O₃, 3H₂O). L'un des facteurs principaux permettant d'orienter cette réaction d'hydrolyse vers une formation préférentielle de l'un de ces oxydes est la quantité d'eau mise en jeu par rapport à la quantité de borate de méthyle. Ainsi, lorsque l'on veut obtenir un produit d'hydrolyse contenant principalement de l'acide métaborique, il est souhaitable d'utiliser une quantité d'eau voisine de 2 moles d'eau par mole de borate de méthyle, par exemple d'environ 1,8 à environ 2,2 moles d'eau par mole de borate de méthyle. Dans le cas où l'on souhaite obtenir un produit contenant une majorité d'anhydride borique, il est souhaitable que la quantité d'eau soit voisine de la quantité théorique, de 1,5 mole d'eau par mole de borate de méthyle, nécessaire pour la formation de ce produit. De même, si la quantité d'eau est voisine ou supérieure à la quantité d'eau théorique, de 3 moles d'eau par mole de borate de méthyle, nécessaire pour la formation de l'acide orthoborique, le produit de l'hydrolyse contiendra en majorité cet acide.

Lors de la réaction d'hydrolyse du borate de méthyle, on utilise le plus souvent une quantité d'eau telle que le rapport molaire eau/borate de méthyle soit généralement d'environ 1,5:1 à environ 2,5:1 et de préférence d'environ 1,8:1 à environ 2,2:1.

Parmi les composés formant avec le méthanol un hétéroazéotrope ayant un point d'ébullition inférieur au point d'ébullition de l'azéotrope méthanol-borate de méthyle, dont une liste non exhaustive peut en particulier être trouvée dans le livre Azeotropic Data, Advances in Chemistry Series 6, American Chemical Society, Washington USA, on choisit de préférence ceux qui forment un hétéroazéotrope contenant au moins 10 % en poids de méthanol dans l'hétéroazéotrope.

Parmi les composés formant avec le méthanol un hétéroazéotrope ayant un point d'ébullition inférieur au point d'ébullition de l'azéotrope méthanol-borate de méthyle, il est également préférable de choisir ceux dont l'hétéroazéotrope a un point d'ébullition inférieur d'au moins 2 °C au point d'ébullition de l'azéotrope méthanol-borate de méthyle.

Le plus souvent, les composés formant ledit hétéroazéotrope sont choisis parmi les hydrocarbures et de préférence les hydrocarbures ayant de 4 à 6 atomes de carbone dans leur molécule et ne comportant pas de cycle à 6 chaînons. A titre d'exemple non limitatif de tels hydrocarbures, on peut citer le méthyl-2 pentane et le diméthyl-2,3 butane.

La quantité du ou des composés utilisés dans la présente invention pour former l'hétéroazéotrope avec le méthanol est habituellement au moins égale à la quantité théorique nécessaire pour enlever azéotropiquement tout le méthanol présent dans la colonne, ainsi que celui qui se forme au cours de l'hydrolyse. La quantité de ce composé sera le plus souvent supérieure à cette quantité théorique et par exemple d'environ 1,1 à environ 2 fois la quantité théorique. Si nécessaire, dans le procédé en continu, la quantité de ce composé sera maintenue sensiblement constante par apport continu ou par bouffées d'une quantité déterminée de ce composé.

Dans une forme avantageuse de réalisation de la présente invention, on alimentera en continu ou par bouffées le décanteur avec une faible proportion d'eau qui permettra une séparation des couches plus facile. La quantité d'eau employée représente habituellement environ 0,5 à environ 10 % en poids, de préférence d'environ 1 à environ 5 % en poids et le plus souvent d'environ 2 à environ 4 % en poids par rapport au poids de méthanol.

Le ou les oxydes qui se forment au cours de l'hydrolyse sont des produits que l'on obtient en fond de colonne sous forme solide extrêmement divisée, habituellement sous forme de sol, en suspension dans le composé de point d'ébullition supérieur à celui du borate de méthyle que l'on a introduit dans ladite colonne. Lorsque l'on effectue l'hydrolyse en continu, on introduit ce composé, en continu ou par bouffées, le plus souvent en quantité équivalente à celle que l'on soutire pour récupérer le ou les oxydes formés. Il est habituel d'opérer de manière à obtenir une suspension contenant de 1 à 50 %, de préférence de 5 à 30 % et le plus souvent de 10 à 20 % en poids de solide par rapport au poids total de la suspension. Ce composé est habituellement introduit dans la colonne à proximité de son fond et de préférence dans le rebouilleur.

Le composé à point d'ébullition supérieur à celui du borate de méthyle que l'on utilise est de préférence un composé dont le point d'ébullition est supérieur d'au moins 2 °C (degrés Celcius), et le plus souvent d'au moins 10 °C, à celui du borate de méthyle. Ce composé est le plus souvent choisi parmi les hydrocarbures et de préférence dans le groupe formé par le cyclohexane et les hydrocarbures saturés ayant de 7 à 20 atomes de carbone dans leur molécule.

Le produit de départ utilisé pour la préparation d'au moins un oxyde de bore dans la présente invention est habituellement choisi dans le groupe formé par le borate de méthyle, l'azéotrope borate de méthyle-méthanol ou un mélange méthanol-borate de méthyle contenant plus d'une mole de méthanol par mole de borate de méthyle. Ce produit de départ peut être le produit résultant de la réaction de l'acide orthoborique, de formule H₃BO₃, sur un excès de méthanol par rapport à la quantité théorique nécessaire à la formation du borate de méthyle, éventuellement en présence d'eau. On utilise habituellement au moins 4 moles de méthanol par mole d'acide orthoborique. Le rapport molaire méthanol : acide orthoborique est par exemple de 4 : 1 à 20 : 1 et de préférence de 5 : 1 à 10 : 1 et le plus souvent on utilise une quantité de méthanol telle que ce rapport soit d'environ 6 : 1. L'utilisation d'un excès de méthanol tel que celui défini ci-avant permet d'obtenir l'azéotrope borate de méthyle-méthanol que l'on récupère en tête d'une colonne à distiller. Cet azéotrope borate de méthyle-méthanol contient 76,5 % en poids de borate de méthyle (soit une mole de borate de méthyle par mole de méthanol). Le borate de méthyle utilisé pourra contenir de l'acide orthoborique non transformé sans inconvénient majeur pour la bonne marche du procédé puisque ce composé sera alors récupéré en fond de colonne, le plus souvent dans le rebouilleur avec le ou les oxydes formés au cours de l'hydrolyse. En opérant avec un excès de méthanol, par exemple dans une colonne réactive de type adiabatique, à une température d'environ 70 °C, la conversion de l'acide orthoborique dépasse 99 % et le plus souvent est supérieure à 99,5 % ; ainsi la quantité d'acide orthoborique non converti que l'on peut récupérer en fond de colonne est donc très faible.

La préparation du borate de méthyle est largement décrite dans l'art antérieur et en particulier dans les brevets US-A-2217354, US-A-2808424, US-A-2813115 et US-A-2947776.

La zone d'hydrolyse du borate de méthyle peut être un réacteur classique, de préférence parfaitement agité, dans lequel l'hydrolyse du borate de méthyle s'effectue, au moins en partie, par exemple jusqu'à l'équilibre correspondant aux diverses conditions employées telles que, par exemple, la quantité d'eau introduite par rapport à la quantité d'acide borique et la température. L'hydrolyse peut également s'effectuer au moins en partie dans la ligne d'alimentation de la colonne à distiller ou encore dans la partie inférieure de la colonne à distiller, par exemple dans le rebouilleur, cette ligne ou cette partie de la colonne formant ladite zone d'hydrolyse et jouant alors le rôle d'un réacteur d'hydrolyse.

Lorsque l'on ne souhaite pas former une majorité d'acide orthoborique, il est souhaitable de choisir des conditions opératoires permettant d'éviter des concentrations locales élevées en eau qui peuvent conduire à sa formation préférentielle et'ou éventuellement de travailler à une température relativement élevée, par exemple d'au moins 120 °C, à laquelle cet acide orthoborique se déshydrate en acide métaborique. L'hydrolyse est le plus souvent effectuée dans des conditions classiques, habituellement à une pression allant de la pression atmosphérique à environ 20 mégapascals (MPa) et le plus souvent d'environ 1,05 à environ 10 MPa et de préférence d'environ 2 à environ 4 MPa. La température à laquelle l'hydrolyse est effectuée est habituellement comprise entre la température ambiante (par exemple environ 18 °C) et environ 200 °C et de préférence, notamment dans le cas où l'on ne désire pas former une majorité d'acide orthoborique, d'environ 100 à environ 180 °C et le plus souvent d'environ 120 à environ 140 °C. Dans tous les cas, l'hydrolyse se poursuit au sein même de la colonne à distiller par déplacement de l'équilibre par suite du départ du méthanol et la conversion molaire du borate de méthyle dépasse le plus souvent 95 %.

La colonne à distiller utilisée dans le cadre de la présente invention est une colonne classique comportant habituellement d'environ 20 à environ 100 plateaux théoriques, le plus souvent d'environ 30 à environ 70 plateaux théoriques et de préférence d'environ 40 à environ 60 plateaux théoriques. Cette colonne, et en particulier le garnissage de la zone d'épuisement, lorsque cette zone on comporte un, est choisie de manière à pouvoir fonctionner, au moins dans la zone d'épuisement, en présence de solide. La zone d'alimentation de la colonne n'est pas très critique. On peut introduire la charge, qui comprend généralement le borate de méthyle et/ou l'azéotrope borate de méthyle-méthanol et de l'eau ou qui comprend le produit issu du réacteur d'hydrolyse, en fond de colonne ou dans le rebouilleur ou dans une zone intermédiaire de la colonne. La charge sera de préférence introduite au-dessus du premier plateau théorique et le plus souvent au-dessus d'une zone constituant une zone d'épuisement de ladite colonne, ladite zone d'épuisement comportant habituellement de 1 à 10 plateaux théoriques et le plus souvent de 2 à 5 plateaux théoriques. Au-dessus de cette zone d'épuisement, la colonne comporte une zone d'enrichissement comprenant habituellement de 20 à 60 plateaux théoriques et le plus souvent de 30 à 50 plateaux théoriques. Le composé de point d'ébullition supérieur à celui du borate de méthyle est habituellement introduit en fond de colonne et par exemple au niveau ou à proximité immédiate du rebouilleur. Le composé formant l'hétéroazéotrope avec le méthanol est habituellement introduit à proximité du sommet de la colonne ou dans le décanteur dans lequel on sépare ce composé du méthanol. Lorsqu'il est nécessaire d'ajouter une certaine quantité de ce composé durant la marche de l'unité, il est alors préférable de l'introduire dans le décanteur.

Les conditions de la distillation sont des conditions classiques aisément déterminables par l'homme du métier en fonction des divers composés présents dans la colonne. On peut ainsi distiller sous une pression inférieure à la pression atmosphérique, à la pression atmosphérique ou sous une pression supérieure à la pression atmosphérique. Le taux de reflux est habituellement d'environ 0,5 à environ 5. La température dans le rebouilleur dépend du composé à point d'ébullition supérieur à celui du borate de méthyle employé dans le procédé. Dans les conditions, notamment de pression, choisies pour la distillation cette température, au niveau du rebouilleur, est habituellement au plus égale à la température d'ébullition de ce composé et supérieure à la température d'ébullition du borate de méthyle ; de préférence cette température sera supérieure d'au moins 10 °C à la température d'ébullition du borate de méthyle.

L'exemple qui suit est donné à titre illustratif, mais nullement limitatif.

### Exemple 1

Dans un réacteur parfaitement agité chauffé à 125 °C et fonctionnant sous une pression absolue de 2 MPa, on injecte en continu 135,8 kg/h (kilogramme par heure) d'un mélange borate de méthyle-méthanol contenant 76,43 % en poids de borate de méthyle (mélange équimolaire) et 36 kg/h d'eau. Le temps de séjour du mélange dans ce réacteur est fixé à 10 minutes. L'effluent du réacteur est introduit en continu dans une colonne à distiller fonctionnant sous pression atmosphérique. Cette colonne comporte 43 plateaux théoriques et l'effluent est introduit au 1/8 de la hauteur totale de la colonne à partir de sa base. On introduit également en fond de colonne et en continu 248,2 kg/h de cyclohexane. Le composé employé pour former un hétéroazéotrope à point d'ébullition inférieur à celui de l'azéotrope borate de méthyle-méthanol est le diméthyl-2,3 butane, qui est introduit dans la colonne au démarrage des opérations. Ce composé forme avec le méthanol un hétéroazéotrope comportant 20 % en poids de méthanol et dont le point d'ébullition est de 45 °C. Dans le condenseur du produit récupéré en tête de la colonne, on introduit en continu 3,8 kg/h d'eau. Le produit condensé est envoyé dans un décanteur dans lequel on récupère une phase lourde contenant l'eau introduite dans le condenseur et pratiquement tout le méthanol produit (résultant de l'hydrolyse du borate de méthyle) et/ou introduit dans la colonne en même temps que l'effluent d'hydrolyse. La quantité de phase lourde récupérée dans le décanteur est de 131,8 kg/h. La phase légère est du diméthyl-2,3 butane contenant des traces de méthanol, elle est renvoyée en tête de colonne à titre de reflux. Le taux de reflux est maintenu constant au cours de l'opération et est égal à 1. On soutire en fond de colonne, à la température de 81 °C, 292 kg/h d'une suspension, dans le cyclohexane, contenant 15 % en poids d'un solide très finement divisé qui est de l'acide métaborique essentiellement pur.

Un autre objet de l'invention est un procédé d'oxydation d'hydrocarbures en les alcools correspondants en présence d'oxyde borique, au cours duquel l'oxyde borique se trouve transformé en acide borique puis en borate de méthyle à partir duquel de l'oxyde borique est régénéré par mise en oeuvre du procédé décrit plus haut.

La fabrication d'alcools par oxydation d'hydrocarbures par un gaz contenant de l'oxygène moléculaire est, depuis de nombreuses années, effectuée en présence d'un composé de bore formant des esters avec l'alcool de manière à augmenter la selectivité en alcool, en limitant l'oxydation ultérieure de l'alcool formé par exemple en composé cétonique.

Ces procédés nécessitent l'hydrolyse des esters boriques formés, de manière à récupérer l'alcool recherché, et pour des raisons économiques, et pour éviter les problèmes de pollution, la récupération et le recyclage de l'acide orthoborique, formé au cours de l'hydrolyse, à l'étape d'oxydation.

De très nombreux brevets concernant ce procédé d'oxydation ont été publiés, en particulier entre 1950 et 1970. Ces documents de brevets mettent en évidence la nécessité de recycler un composé de bore aussi pur que possible.

En effet, comme cela est en particulier mentionné dans les brevets français FR-B-1497519 et FR-B-1498351 au nom de la demanderesse, il est souhaitable d'éviter le recyclage des diverses impuretés organiques avec l'acide borique, car ces impuretés inhibent la réaction d'oxydation et conduisent à un encrassement du réacteur.

Les procédés décrits dans ces brevets impliquent, pour la récupération de l'acide borique, en vu de son recyclage, une étape de cristallisation de l'acide borique par refroidissement de la phase aqueuse issue de la zone d'hydrolyse et une étape de lavage par un solvant, tel que le méthanol ou un mélange méthanol et eau, de l'acide cristallisé, ainsi qu'éventuellement une étape de séchage ou de déshydratation de l'acide borique avant son recyclage à l'étape d'oxydation de l'hydrocarbure.

Ces procédés présentent plusieurs inconvénients, dont les plus importants sont liés à l'utilisation d'équipements spécifiques utilisés dans l'industrie minérale tels que cristalliseurs, centrifugeuses et fours rotatifs, dont la mise en oeuvre est délicate et malaisée, et dont le coût est important. Par ailleurs, l'emploi d'un solvant de lavage tel que le méthanol entraîne une perte importante de composé de bore qui est plus ou moins soluble dans cet alcool et qui de plus risque de réagir avec lui pour former du borate de méthyle soluble dans le méthanol. Il faut enfin mentionner que les cristaux de composé borique, obtenus au cours de ces procédés, sont relativement gros, ce qui limite leur réactivité vis-à-vis de l'alcool qui se forme dans l'étape d'oxydation et donc diminue la sélectivité en alcool de l'ensemble du procédé.

Le procédé décrit dans la demande de brevet français FR-A-2363516 publiée en 1978 présente, globalement, les mêmes inconvénients que ceux mentionnés ci-avant pour les procédés décrits par la demanderesse, et en particulier ceux liés à l'utilisation d'équipements spécifiques de l'industrie minérale.

Il est connu et décrit, notamment dans le brevet FR-B-1513001, que le traitement de séchage et de déshydratation a également pour but de transformer au moins en partie l'acide orthoborique en un acide de degré d'hydratation inférieur, notamment en acide métaborique, qui recyclé à l'étape d'oxydation permet l'obtention d'un meilleur rendement en alcool.

Ainsi, bien que l'on puisse utiliser dans le processus d'oxydation aussi bien l'acide orthoborique, l'acide métaborique, l'anhydride borique et les mélanges de ces composés de bore comme cela est en particulier décrit dans la demande de brevet français FR-A-2209737 page 1 ligne 14 à 21, il est cependant préférable, d'après l'enseignement de cette demande de brevet, de transformer au moins en partie l'acide orthoborique en acide métaborique que l'on renvoie à la zone d'oxydation (page 5, lignes 14 à 17).

Il est à souligner que l'enseignement de ces deux documents de brevets, FR-B-1513001 et FR-A-2209737, ne permet pas de pallier les inconvénients mentionnés ci-avant et en particulier ceux liés à l'emploi d'équipements spécifiques de l'industrie minérale.

La présente invention propose une solution technique, complètement différente de celle utilisée depuis plus de 25 ans, qui permet de limiter au minimum l'utilisation d'équipements spécifiques de l'industrie minérale et même, dans sa forme préférée de mise en oeuvre, de ne pas avoir à utiliser de tels équipements.

Le procédé de la présente invention n'utilise, dans sa forme la plus large, pour ce qui est de la partie concernant le recyclage du composé de bore, qu'un seul équipement spécifique de l'industrie minérale qui est un équipement permettant de récupérer l'acide borique à partir de son mélange avec un composé à point d'ébullition plus élevé que celui du borate de méthyle. Le plus souvent, l'oxyde borique à récupérer est en suspension dans un solvant organique et on utilise alors une simple centrifugeuse ou un simple appareil de filtration.

Dans sa forme préférée de mise en oeuvre, le procédé de la présente invention, pour ce qui est de la partie concernant le recyclage du composé de bore, ne fait appel qu'à des opérations de distillation, de mise en oeuvre aisée et de coût de maintenance et de main-d'oeuvre moins important que lorsque on utilise des équipements spécifiques de l'industrie minérale. En effet, dans cette forme préférée, le composé de bore est obtenu en suspension dans l'hydrocarbure que l'on oxyde et il est alors possible de recycler cette suspension sans avoir à effectuer de séparation du composé de bore.

Le procédé de la présente invention permet de recycler un composé de bore contenant moins d'impuretés, en particulier d'impuretés organiques, que le composé recyclé selon les techniques antérieures tout en ayant une perte de bore extrêmement limitée. Il est également de mise en oeuvre aisée et, par ailleurs, il permet d'obtenir un composé de bore sous une forme extrêmement divisée, le plus souvent au moins en partie sous la forme d'un sol, ce qui permet d'obtenir une sélectivité en alcool plus élevée que celle obtenue selon la technique antérieure et relativement constante au cours du temps.

De façon plus précise, la présente invention concerne un procédé d'oxydation d'au moins un hydrocarbure saturé choisi dans le groupe formé par le cyclohexane et les hydrocarbures saturés acycliques et cycliques ayant de 7 à 20 atomes de carbone dans leur molécule, en un produit comprenant l'alcool correspondant, selon lequel :
a) on oxyde ledit hydrocarbure, en phase liquide, à l'aide d'un gaz contenant de l'oxygène moléculaire, en présence d'au moins un composé de bore, formant des esters avec l'alcool formé au cours de l'oxydation, choisi dans le groupe formé par les oxydes boriques, sous forme anhydre ou hydratée, de formule générale

   (I) B₂O₃,xH₂O

   dans laquelle x est un nombre de 0 à 3,
b) on hydrolyse le mélange réactionnel obtenu à l'étape a), contenant au moins un ester dudit composé de bore, en un produit comprenant de l'acide orthoborique et un alcool correspondant audit hydrocarbure,
c) on sépare le mélange réactionnel obtenu à l'étape b) en une phase aqueuse contenant de l'acide orthoborique et en une phase organique, contenant un alcool correspondant audit hydrocarbure, à partir de laquelle on récupère ledit alcool, ledit procédé étant caractérisé en ce qu'il comporte les étapes suivantes :
d) on traite, dans des conditions de formation du borate de méthyle, la phase aqueuse obtenue à l'étape c) par du méthanol en une quantité molaire au moins égale à la quantité théorique nécessaire à la formation de l'azéotrope borate de méthyle-méthanol, et on sépare, dans une colonne à distiller, le borate de méthyle sous la forme de son azéotrope avec le méthanol, azéotrope que l'on récupère en tête de colonne, et une phase, à point d'ébullition plus élevé que celui de l'azéotrope méthanol-borate de méthyle, contenant de l'eau, que l'on récupère en fond de colonne,
e) on introduit dans une zone d'hydrolyse l'azéotrope méthanol-borate de méthyle récupéré à l'étape d) et de l'eau, dans un rapport molaire eau/borate de méthyle d'environ 1,3 : 1 à environ 2,9 : 1, et on effectue, dans ladite zone, au moins en partie l'hydrolyse du borate de méthyle,
f) on introduit dans une colonne à distiller :
   - le produit issu de la zone d'hydrolyse de l'étape e)
   - au moins un composé formant un hétéroazéotrope avec le méthanol, ledit hétéroazéotrope ayant un point d'ébullition inférieur à celui de l'azéotrope méthanol-borate de méthyle, et
   - au moins un composé ayant un point d'ébullition supérieur à celui du borate de méthyle, ledit composé ne formant pas d'azéotrope à point d'ébullition inférieur à celui dudit hétéroazéotrope,
g) on récupère, en tête de la colonne employée à l'étape f), ledit hétéroazéotrope que l'on envoie dans un décanteur dans lequel on sépare le méthanol du composé formant ledit hétéroazéotrope et on recycle ce composé dans la colonne à distiller à titre de reflux et, au moins en partie, à titre d'alimentation de ladite colonne en ce composé,
h) on récupère, en fond de la colonne employée à l'étape f), un mélange comprenant au moins un oxyde borique, sous forme anhydre ou hydratée, et ledit composé à point d'ébullition supérieur à celui du borate de méthyle, et
i) on recycle, à l'étape a), le mélange récupéré à l'étape h) ou ledit oxyde borique obtenu par séparation à partir dudit mélange récupéré à l'étape h).

Les étapes a) d'oxydation, b) d'hydrolyse et c) de séparation d'une phase organique et d'une phase aqueuse sont des étapes classiques dont les conditions générales de mise en oeuvre sont bien connues de l'homme du métier. Ces conditions ne seront pas détaillées dans le cadre de la de la présente description. De très nombreux documents mentionnent ces conditions tels que par exemple Ullmann's Encyclopedia of Industrial Chemistry, Fifth, completely Revised Edition, Vol. A8, pages 220 et 221, Benzene and its derivatives, edited by E. G. HANCOCK, LONDON 1975, pages 238 à 251, et les documents de brevets FR-B-1442272, FR-B-1497522, FR-B-1536937, FR-B-1549178, FR-B-1556968, FR-B-1524498, FR-B-1556980, US-A-3895067 et US-A-3932513.

Dans l'étape a) d'oxydation, on emploie habituellement un gaz contenant de 2 à 25 % en volume d'oxygène, par exemple un mélange d'azote et d'oxygène, de l'air, éventuellement dilué par un gaz inerte tel que l'azote, mais il est également possible d'employer un gaz plus riche en oxygène tel que de l'air enrichi en oxygène. L'oxydation est effectuée dans des conditions de température et de pression telles qu'elle se déroule en phase liquide. La température d'oxydation est généralement d'environ 100 à 250 °C et le plus souvent d'environ 130 à 200 °C et la pression est habituellement d'environ 0,5 à 4 Mégapascals (MPa).

Au cours de l'étape a) d'oxydation, le composé de bore utilisé est de préférence choisi dans le groupe formé par l'anhydride borique de formule générale (I) dans laquelle x est égal à zéro, l'acide métaborique de formule générale (I) dans laquelle x est égal à 1, les mélanges de ces deux composés et les mélanges d'acide métaborique et d'acide orthoborique contenant moins de 60 % et de préférence moins de 30 % en poids d'acide orthoborique de formule générale (I) dans laquelle x est égal à 3.

Lorsque le composé de bore utilisé dans l'étape d'oxydation est l'un des composés préférés mentionnés ci-avant, il est alors nécessaire pour la bonne marche du procédé de choisir les conditions de l'hydrolyse du borate de méthyle à l'étape e) de sorte que le produit obtenu soit celui que l'on souhaite, c'est-à-dire l'un des composés préférés mentionnés ci-avant.

Dans l'étape b) d'hydrolyse du mélange réactionnel, issu de l'étape a) d'oxydation, on emploie habituellement une quantité d'eau d'environ 0,01 à environ 1 fois, en poids, le poids dudit effluent réactionnel, la température de l'hydrolyse est habituellement d'environ 20 à environ 170 °C et la pression est d'environ 0,1 à environ 4 MPa. Cette réaction d'hydrolyse est très rapide, sa durée est généralement d'environ 5 minutes à environ 4 heures.

Dans l'étape c), la séparation entre une phase organique et une phase aqueuse est effectuée dans des conditions classiques, par exemple dans un décanteur. La phase organique, contenant l'alcool formé au cours de l'oxydation, est ensuite envoyée dans une section de traitement et de récupération dudit alcool. Ce traitement comprend de préférence une première étape de lavage à l'eau qui est habituellement suivie par une étape de saponification et éventuellement une deuxième étape de lavage à l'eau, puis l'alcool est récupéré par exemple par distillation ou par extraction à l'aide d'un solvant. Ce traitement de la phase organique est un traitement classique décrit par exemple dans plusieurs des documents cités ci-avant. L'eau de lavage utilisée dans la première étape de lavage est de préférence au moins en partie utilisée ensuite à l'étape b) d'hydrolyse du mélange réactionnel obtenu à l'étape a). Cette eau de lavage peut comprendre, dans une forme préférée de réalisation, au moins une partie de l'eau séparée en fond de colonne à l'étape d) de formation du borate de méthyle.

L'étape d) de formation de borate de méthyle et de séparation, dans une colonne à distiller (comprenant dans sa partie inférieure une zone d'épuisement et dans sa partie supérieure une zone d'enrichissement), de l'azéotrope borate de méthyle-méthanol en tête de colonne et en fond de colonne d'une phase à point d'ébullition plus élevé, contenant de l'eau, peut être effectuée dans deux zones distinctes (un réacteur d'estérification et une colonne à distiller) ou dans une seule et même zone de distillation-réaction dans laquelle la réaction de formation du borate de méthyle se déroule simultanément à la séparation de l'azéotrope que l'on récupère en tête de ladite zone. On utilise habituellement au moins 4 moles de méthanol par mole d'acide orthoborique. Le rapport molaire méthanol : acide orthoborique est par exemple de 4 : 1 à 20 : 1 et de préférence de 5 : 1 à 10 : 1 et le plus souvent on utilise une quantité de méthanol telle que ce rapport soit d'environ 6 : 1. L'utilisation d'une quantité de méthanol au moins égale à la quantité théorique nécessaire à la formation de l'azéotrope borate de méthyle-méthanol (soit 4 moles de méthanol par mole d'acide orthoborique), ou d'un excès de méthanol tel que celui défini ci-avant, permet d'obtenir l'azéotrope borate de méthyle-méthanol que l'on récupére en tête d'une colonne à distiller. Cet azéotrope borate de méthyle-méthanol contient 76,5 % en poids de borate de méthyle (soit une mole de borate de méthyle par mole de méthanol).

Cette colonne à distiller peut travailler à une pression inférieure, égale ou supérieure à la pression atmosphérique. On préfère habituellement effectuer cette distillation à pression atmosphérique avec une température en fond de colonne d'environ 70 °C à environ 100 °C. Dans ces conditions, en opérant avec un excès de méthanol, par exemple dans une colonne réactive de type adiabatique, la conversion de l'acide orthoborique dépasse 99 % et le plus souvent est supérieure à 99,5 % ; ainsi la quantité d'acide orthoborique non converti que l'on récupère en fond de colonne est très faible.

Dans une forme avantageuse de réalisation de l'invention, les conditions de la distillation sont choisies de manière à obtenir en fond de colonne un produit à point d'ébullition plus élevé que celui de l'azéotrope borate de méthyle-méthanol, contenant de l'eau et une très faible quantité de méthanol, de préférence inférieure à 1 % et le plus souvent inférieure à 0,2 % en poids par rapport au poids de ce produit. Ceci peut être obtenu en opérant sous une pression donnée à une température, en fond de colonne, supérieure à la température d'ébullition du méthanol.

Dans ces conditions, le méthanol en excès est par exemple soutiré de la colonne dans une zone médiane de la zone d'enrichissement, puis recyclé soit dans le réacteur de formation du borate de méthyle, soit dans la colonne elle-même, par exemple au niveau de l'introduction de la charge comprenant l'acide orthoborique et l'eau en provenance de l'étape c) et du méthanol. Il est également possible de soutirer le méthanol en excès, en partie ou en totalité, avec l'azéotrope borate de méthyle-méthanol et d'envoyer ce mélange à l'étape e) d'hydrolyse du borate de méthyle, puis le produit résultant à la colonne de distillation de l'étape f) dans laquelle tout le méthanol est séparé en tête sous forme d'un hétéroazéotrope. Le plus souvent, au moins une majeure partie du méthanol en excès est soutiré dans la zone médiane de la zone d'enrichissement de la colonne. Cette colonne à distiller employée à l'étape d) est une colonne classique comportant habituellement d'environ 20 à 100 plateaux théoriques et le plus souvent d'environ 30 à 80 plateaux théoriques. La zone d'épuisement de cette colonne comporte habituellement de 1 à 20 plateaux théoriques et le plus souvent de 2 à 10 plateaux théoriques. Au dessus de cette zone d'épuisement, la zone d'enrichissement comprend habituellement de 20 à 80 plateaux théoriques et le plus souvent de 30 à 70.

Dans une forme préférée de réalisation de l'invention, le méthanol, séparé dans le décanteur au cours de l'étape g), est recyclé à l'étape d) de formation du borate de méthyle.

Dans une forme particulièrement avantageuse de réalisation de l'invention, le composé à point d'ébullition supérieur à celui du borate de méthyle que l'on utilise est l'hydrocarbure soumis à l'oxydation à l'étape a).

Le ou les oxydes boriques, sous forme anhydre ou hydratée, de formule générale (I) ci-avant, qui se forment au cours de l'hydrolyse, à l'étape e), sont des produits que l'on obtient à l'étape h), en fond de la colonne employée à l'étape f), en mélange avec le composé à point d'ébullition supérieur à celui du borate de méthyle, le plus souvent sous forme d'une suspension dans ce composé, sont obtenus à partir du borate de méthyle avec une très bonne conversion habituellement supérieure à 95 % en moles et le plus souvent d'environ 100 %, sous la forme d'un produit extrêmement divisé et relativement pur. Le mélange obtenu à l'étape h) peut être envoyé dans une zone de séparation dans laquelle on récupère le ou les oxydes boriques solides que l'on recycle à l'étape a). Dans une forme préférée de réalisation de l'invention, le ou les oxydes boriques solides, sont obtenus sous forme d'une suspension de solide extrêmement divisé, habituellement au moins en partie sous forme de sol, dans l'hydrocarbure soumis à l'oxydation, et dans ce cas on ne sépare pas ce ou ces oxydes boriques et on recycle cette suspension à l'étape a).

L'invention s'applique particulièrement bien au cas de l'oxydation du cyclohexane en un produit d'oxydation contenant du cyclohexanol le plus souvent en quantité majoritaire par rapport aux autres produits d'oxydation.

L'exemple qui suit illustre le second objet de l'invention.

### Exemple 2

Dans une unité fonctionnant en continu, on oxyde du cyclohexane liquide au moyen d'un mélange gazeux d'oxygène et d'azote renfermant en volume 4 % d'oxygène. Ce mélange gazeux est introduit par la ligne (40) dans le cyclohexane liquide, porté à la température de 165 °C, contenu dans le réacteur (A) et contenant en suspension des particules d'acide métaborique essentiellement pur introduit également en continu dans le réacteur d'oxydation. La pression opératoire est de 1 MPa. Les débits de cyclohexane et d'acide métaborique essentiellement pur introduits dans le réacteur (A) sont respectivement de 83 tonnes par heures (t/h) et de 3,7 t/h. Le cyclohexane est introduit par la ligne (150) à raison de 62 t/h et par la ligne (19) à raison de 21 t/h. Le temps de séjour dans le réacteur d'oxydation est de 2 heures et le taux de conversion du cyclohexane est de 12 %.

L'effluent d'oxydation est mis en contact avec l'eau nécessaire à l'hydrolyse dans la ligne (1) et l'ensemble est introduit dans le réacteur (B) d'hydrolyse. L'eau nécessaire pour effectuer l'hydrolyse provient par la ligne (2) de la colonne de lavage (C1) de la phase organique. La quantité d'eau introduite par la ligne (2) est de 7,4 t/h soit un poids représentant 0,089 fois le poids de l'effluent d'oxydation. Le temps de séjour dans le réacteur d'hydrolyse est de 10 minutes, la température est de 145 °C et la pression de 1 MPa. La selectivité molaire en cyclohexanol est de 83 %.

L'effluent du réacteur (B) d'hydrolyse est envoyé par la ligne (3) dans le décanteur (B1) dans lequel on sépare une phase organique légère, contenant une faible quantité d'acide orthoborique, d'une phase aqueuse lourde contenant en solution la majeure partie de l'acide orthoborique présent dans l'effluent du réacteur (B). La phase organique légère est envoyée par la ligne (4) dans une colonne de lavage à l'eau (C1) dans laquelle l'eau de lavage, contenant des traces d'acide orthoborique, en provenance de la colonne de distillation du borate de méthyle (C2), est introduite par la ligne (8). Au cours de ce lavage, l'acide orthoborique présent dans la phase organique est extrait par l'eau de lavage et la phase aqueuse lourde récupérée en fond de la colonne de lavage (C1) est envoyée à l'étape d'hydrolyse par la ligne (2). La phase organique légère récupérée en tête de la colonne de lavage (C1) est envoyée vers la section de purification et de récupération du cyclohexanol par la ligne (6).

La phase aqueuse lourde, contenant en solution la majeure partie de l'acide orthoborique présent dans l'effluent du réacteur (B), est récupérée en fond du décanteur (B1) par la ligne (5). Cette phase aqueuse, qui contient, outre l'acide orthoborique à recycler, des produits solubles et en particulier du cyclohexanol, de la cyclohexanone, des acides et d'autres sous-produits organiques formés au cours de l'oxydation, est mise en contact dans la ligne (9) avec le méthanol nécessaire à la formation du borate de méthyle. La quantité de méthanol introduite dans la ligne (9) par la ligne (17) est de 11 t/h. Ce méthanol provient du décanteur (D) de la colonne de distillation (C3) du mélange hétéroazéotropique de diméthyl-2,3 butane et de méthanol. Le produit contenu dans la ligne (9) est mélangé avec le méthanol soutiré de la colonne (C2) par la ligne (10) et l'ensemble, formant la charge pour la préparation du borate de méthyle, est introduit dans la colonne de distillation réactive (C2) par la ligne (11). Cette colonne de distillation réactive comporte 65 plateaux théoriques. La charge est introduite dans la colonne (C2) au niveau du 25e plateau (compter à partir du bas) et le courant latéral de méthanol est soutiré au niveau du 55e plateau. Le taux de reflux de la colonne (C2) est fixé à 1,2 et on distille en tête à la température de 54,6 °C 11,47 t/h d'azéotrope borate de méthyle-méthanol que l'on envoie par la ligne (12) dans le réacteur d'hydrolyse (R1). On soutire en fond de colonne 10,34 t/h d'un produit essentiellement aqueux contenant la totalité des produits organiques solubles dans l'eau, des traces de méthanol et 0,21 % en poids d'acide orthoborique non estérifié.

La majeure partie de cette phase aqueuse, soutirée en fond de la colonne (C2), est envoyée par la ligne (8) à la colonne de lavage (C1), le reste est évacué par la ligne (20). La quantité de produit rejeté par la ligne (20) est de 3,3 t/h.

Dans le réacteur d'hydrolyse parfaitement agité (R1), on introduit par la ligne (12) l'azéotrope borate de méthyle-méthanol et la quantité stoechiométrique d'eau nécessaire à la formation de l'acide métaborique par la ligne (14), soit 3,04 t/h. Dans le réacteur (R1) parfaitement agité, l'hydrolyse du borate de méthyle est effectuée à la température de 125 °C sous une pression absolue de 2 MPa. Le temps de séjour du mélange borate de méthyle-méthanol-eau dans le réacteur (R1) est fixé à 10 minutes.

L'effluent du réacteur (R1) est introduit en continu par la ligne (60) dans la colonne à distiller (C3) fonctionnant sous pression atmosphérique. Cette colonne comporte 43 plateaux théoriques et l'effluent est introduit au 1/8 de la hauteur totale de la colonne à partir de sa base. On introduit également dans cette colonne (C3), par la ligne (15), au même niveau que l'effluent du réacteur (R1) et en continu, 21 t/h de cyclohexane. Au démarrage des opérations on introduit dans la colonne (C3) 10 tonnes de diméthyl-2,3 butane à titre de composé formant, avec le méthanol, l'hétéroazéotrope à point d'ébullition inférieur à celui de l'azéotrope borate de méthyle-méthanol. Ce composé forme avec le méthanol un hétéroazéotrope comportant 20 % en poids de méthanol et dont le point d'ébullition est de 45 °C. Dans le décanteur du produit récupéré en tête de la colonne, on introduit en continu par la ligne (50) 0,32 t/h d'eau (soit 3 % en poids par rapport au poids de méthanol). Le produit récupéré en tête de colonne par la ligne (16) est condensé, puis envoyé dans le décanteur (D) dans lequel on récupère une phase lourde contenant l'eau introduite par la ligne (50) et pratiquement tout le méthanol formé par hydrolyse du borate de méthyle. La quantité de phase lourde récupérée dans le décanteur par la ligne (17) est de 11 t/h. La phase légère est du diméthyl-2,3 butane contenant des traces de méthanol, elle est renvoyée par la ligne (18) en tête de la colonne (C3) à titre de reflux à raison de 43,2 t/h. Le taux de reflux est maintenu constant au cours de l'opération et est égal à 1. On soutire en fond de la colonne (C3), à la température de 81 °C, 24,77 t/h d'une suspension dans le cyclohexane de 15 % en poids d'un solide très finement divisé qui est de l'acide métaborique essentiellement pur. Cette suspension est recyclée par la ligne (19) au réacteur d'oxydation (A).

Après une durée de fonctionnement de 5000 heures, l'unité est arrêtée et les appareils sont inspectés. On ne constate pas d'accumulation de dépôts susceptible de mettre en cause leur fonctionnement. Au cours du test, la sélectivité en cyclohexanol est restée sensiblement constante et la perte horaire en composé borique est inférieure à 5 kilogrammes par heure.

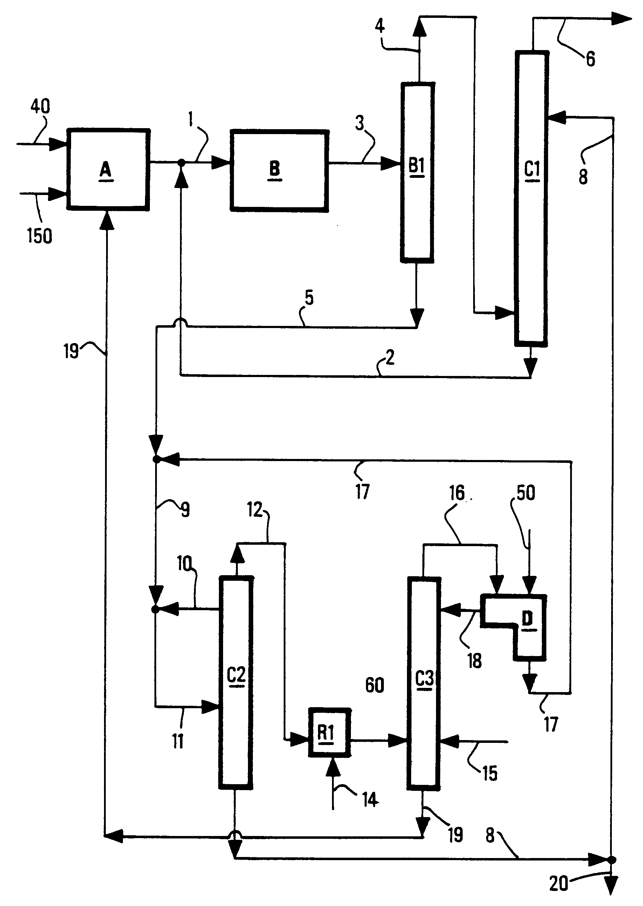

## Revendications

1. Procédé de préparation d'au moins un oxyde borique, sous forme anhydre ou hydratée, de formule générale
(I) B₂O₃,xH₂O
dans laquelle x est un nombre de 0 à 3, par hydrolyse de borate de méthyle dans lequel on introduit, dans une zone d'hydrolyse, du borate de méthyle et de l'eau dans un rapport molaire d'au moins 1,3 mole d'eau par mole de borate de méthyle, puis on introduit dans une colonne à distiller le produit issu de la zone d'hydrolyse, comprenant ledit oxyde borique et du méthanol, et au moins un compose formant un hétéroazéotrope avec le méthanol, ledit hétéroazéotrope ayant un point d'ébullition inférieur à celui de l'azéotrope borate de méthyle-;méthanol, caractérisé en ce que :
- on introduit également dans ladite colonne au moins un composé ayant un point d'ébullition supérieur à celui du borate de méthyle, ledit composé ne formant pas d'azéotrope à point d'ébullition inférieur à celui dudit hétéroazéotrope,
- on récupère, en tête de colonne, ledit hétéroazéotrope que l'on envoie dans un décanteur dans lequel on sépare le méthanol du composé formant ledit hétéroazéotrope et on recycle ce composé dans la colonne à distiller à titre de reflux et d'alimentation de ladite colonne en ce composé, et,
- on récupère, en fond de colonne, un mélange d'au moins un oxyde borique, sous forme anhydre ou hydratée, et du composé à point d'ébullition supérieur à celui du borate de méthyle.

2. Procédé selon la revendication 1 dans lequel le composé formant un hétéroazéotrope avec le méthanol est choisi dans le groupe des composés formant un hétéroazéotrope contenant au moins 10% en poids de méthanol dans l'hétéroazéotrope.

3. Procédé selon la revendication 1 ou 2 dans lequel le composé formant un hétéroazéotrope avec le méthanol est choisi parmi ceux dont l'hétéroazéotrope a un point d'ébullition inférieur d'au moins 2 °C au point d'ébullition de l'azéotrope méthanol-borate de méthyle.

4. Procédé selon l'une des revendications 1 à 3 dans lequel le composé formant un hétéroazéotrope avec le méthanol est choisi parmi les hydrocarbures.

5. Procédé selon l'une des revendications 1 à 4 dans lequel le composé formant un hétéroazéotrope avec le méthanol est choisi dans le groupe formé par les hydrocarbures ayant 4 à 6 atomes de carbone dans leur molécule et ne comportant pas de cycle à 6 chaînons.

6. Procédé selon l'une des revendications 1 à 5 dans lequel le composé formant un hétéroazéotrope avec le méthanol est choisi dans le groupe formé par le méthyl-2 pentane et le diméthyl-2,3 butane.

7. Procédé selon l'une des revendications 1 à 6 dans lequel le composé à point d'ébullition supérieur à celui du borate de méthyle est choisi parmi les composés ayant un point d'ébullition supérieur d'au moins 2 °C à celui du borate de méthyle.

8. Procédé selon l'une des revendications 1 à 7 dans lequel le composé à point d'ébullition supérieur à celui du borate de méthyle est choisi parmi les hydrocarbures.

9. Procédé selon l'une des revendications 1 à 8 dans lequel le composé à point d'ébullition supérieur à celui du borate de méthyle est choisi dans le groupe formé par le cyclohexane et les hydrocarbures saturés ayant de 7 à 20 atomes de carbone dans leur molécule.

10. Procédé selon l'une des revendications 1 à 9 dans lequel on introduit dans le décanteur de l'eau en quantité d'au moins 0,5 % en poids par rapport au poids de méthanol présent.

11. Procédé selon l'une des revendications 1 à 10 dans lequel on utilise comme produit de départ, pour la préparation d'au moins un oxyde borique, sous forme anhydre ou hydratée, le borate de méthyle, l'azéotrope méthanol-borate de méthyle ou un mélange méthanol-borate de méthyle contenant plus d'une mole de méthanol par mole de borate de méthyle.

12. Procédé selon l'une des revendications 1 à 11 dans lequel le borate de méthyle utilisé comme produit de départ pour la préparation d'au moins un oxyde borique, sous forme anhydre ou hydratée, est le produit résultant de la réaction de l'acide orthoborique, de formule H₃BO₃, sur un excès de méthanol, par rapport à la quantité théorique nécessaire à la formation du borate de méthyle, éventuellement en présence d'eau.

13. Procédé d'oxydation d'au moins un hydrocarbure saturé, choisi dans le groupe formé par le cyclohexane et les hydrocarbures saturés acycliques et cycliques ayant de 7 à 20 atomes de carbone dans leur molécule, en un produit comprenant l'alcool correspondant, selon lequel :
a) on oxyde ledit hydrocarbure, en phase liquide, à l'aide d'un gaz contenant de l'oxygène moléculaire, en présence d'au moins un composé de bore, formant des esters avec l'alcool formé au cours de l'oxydation, choisi dans le groupe formé par les oxydes boriques, sous forme anhydre ou hydratée, de formule générale
(I) B₂O₃,xH₂O
dans laquelle x est un nombre de 0 à 3,
b) on hydrolyse le mélange réactionnel obtenu à l'étape a), contenant au moins un ester dudit composé de bore, en un produit oomprenant de l'acide orthoborique et un alcool correspondant audit hydrocarbure,
c) on sépare le mélange réactionnel obtenu à l'étape b) en une phase aqueuse contenant de l'acide orthoborique et en une phase organique, contenant un alcool correspondant audit hydrocarbure, à partir de laquelle on récupère ledit alcool,
ledit procédé incluant la régénération d'oxyde borique par un procédé selon l'une des revendications 1 à 12 étant caractérisé en ce qu'il comporte les étapes suivantes :
d) on traite, dans des conditions de formation du borate de méthyle, la phase aqueuse obtenue à l'étape c) par du méthanol an une quantité molaire au moins égale à la quantité théorique nécessaire à la formation de l'azéotrope borate de méthyle-méthanol, et on sépare, dans une colonne à distiller, le borate de méthyle sous la forme de son azéotrope avec le méthanol, azéotrope que l'on récupère en tête de colonne, et une phase, à point d'ébullition plus élevé que celui de l'azéotrope méthanol-borate de méthyle, contenant de l'eau, que l'on récupère en fond de colonne,
e) on introduit dans une zone d'hydrolyse l'azéotrope méthanol-borate de méthyle récupéré à l'étape d) et de l'eau, dans un rapport molaire eau/borate de méthyle de 1,3 : 1 à 2,9 : 1, et on effectue, dans ladite zone, au moins en partie l'hydrolyse du borate de méthyle,
f) on introduit dans une colonne à distiller :
- le produit issu de la zone d'hydrolyse de l'étape e)
- au moins un composé formant un hétéroazéotrope avec le méthanol, ledit hétéroazéotrope ayant un point d'ébullition inférieur à celui de l'azéotrope méthanol-borate de méthyle, et
- au moins un composé ayant un point d'ébullition supérieur à celui du borate de méthyle ledit composé ne formant pas d'azéotrope à point d'ébullition inférieur à celui dudit hétéroazéotrope,
g) on récupère, en tête de la colonne employée à l'étape f), ledit hétéroazéotrope que l'on envoie dans un décanteur dans lequel on sépare le méthanol du composé formant ledit hétéroazéotrope et on recycle ce composé dans la colonne à distiller à titre de reflux et, au moins en partie, à titre d'alimentation de ladite colonne en ce composé,
h) on récupère, en fond de la colonne employée à l'étape f), un mélange comprenant au moins un oxyde borique, sous forme anhydre ou hydratée, et ledit composé à point d'ébullition supérieur à celui du borate de méthyle, et
i) on recycle, à l'étape a), le mélange récupéré à l'étape h) ou ledit oxyde borique obtenu par séparation à partir dudit mélange récupéré à l'étape h).

14. Procédé selon la revendication 13 dans lequel le composé de bore est choisi dans le groupe formé par l'anhydride borique de formule générale (I) dans laquelle x est égal à zéro, l'acide métaborique de formule générale (I) dans laquelle x est égal à 1, les mélanges de ces deux composés et les mélanges d'acide métaborique et d'acide orthoborique contenant moins de 60 % en poids d'acide orthoborique de formule générale (I) dans laquelle x est égal à 3, dans lequel à l'étape e) le rapport molaire eau/borate de méthyle est d'environ 1,5 : 1 à environ 2,6 : 1 et dans lequel les conditions de l'hydrolyse sont telles que l'on forme soit de l'anhydride borique, soit de l'acide métaborique, soit un mélange de ces deux composés, soit un mélange comprenant de l'acide métaborique et de l'acide orthoborique ledit mélange contenant moins de 60 % en poids d'acide orthoborique.

15. Procédé selon la revendication 13 ou 14 dans lequel l'étape d) est effectuée dans un zone de réaction-distillation.

16. Procédé selon l'une des revendications 13 à 15 dans lequel le méthanol séparé à l'étape g) est recyclé à l'étape d) de formation du borate de méthyle.

17. Procédé selon l'une des revendications 13 à 16 dans lequel la phase organique séparée à l'étape c) est soumise à un lavage à l'eau avant la récupération de l'alcool, et l'eau de lavage est au moins en partie utilisée à l'étape b) d'hydrolyse du mélange réactionnel obtenu à l'étape a).

18. Procédé selon la revendication 17 dans lequel l'eau employée pour le lavage de la phase organique séparée à l'étape c) provient au moins en partie de l'étape d) de formation du borate de méthyle.

19. Procédé selon l'une des revendications 13 à 18 dans lequel l'hydrocarbure saturé est le cyclohexane.

20. Procédé selon l'une des revendications 13 à 19 dans lequel le composé à point d'ébullition supérieur à celui du borate de méthyle employé à l'étape f) est l'hydrocarbure soumis à l'oxydation à l'étape a).

## Claims

1. Process for the preparation of at least one boric oxide, in anhydrous or hydrated form and of general formula
(I) B₂O₃, xH₂O,
in which x is number from 0 to 3, by hydrolysis of methyl borate, in which is introduced in a hydrolysis zone methyl borate and water in a molar ratio of at least 1.3 mole of water per mole of methyl borate and then into a distillation column is introduced the product from the hydrolysis zone, incorporating the boric oxide and methanol, and at least one compound forming a heteroazeotrope with methanol, said heteroazeotrope having a boiling point below that of the methyl borate-methanol azeotrope, characterized in that:
- into the said column is also introduced at least one compound having a boiling point above that of methyl borate, said compound not forming an azeotrope with a boiling point below that of said heteroazeotrope,
- at the top of the column the said heteroazeotrope is recovered and fed into a decanter, in which the methanol is separated from the compound forming said heteroazeotrope and said compound is recycled into the distillation column as reflux and as a charge for said column and
- at the bottom of the column recovery takes place of a mixture of at least one boric oxide, in anhydrous or hydrated form, and the compound having a boiling point higher than that of methyl borate.

2. Process according to claim 1, wherein the compound forming a heteroazeotrope with methanol is selected from the group of compounds forming a heteroazeotrope containing at least 10% by weight methanol in the heteroazeotrope.

3. Process according to claim 1 or 2, wherein the compound forming a heteroazeotrope with methanol is selected from among those whose heteroazeotrope has a boiling point lower by at least 2°C than the boiling point of the methanol-methyl borate azeotrope.

4. Process according to anyone of claims 1 to 3 wherein the compound forming a heteroazeotrope with methanol is selected from among the hydrocarbons.

5. Process according to anyone of claims 1 to 4 wherein the compound forming a heteroazeotrope with methanol is selected from the group consisting of hydrocarbons having 4 to 6 carbon atoms in their molecule and no 6 groups cycle.

6. Process according to anyone of claims 1 to 5 wherein the compound forming a heteroazeotrope with methanol is selected from the group consisting of 2-methyl pentane and 2,3-dimethyl butane.

7. Process according to anyone of claims 1 to 6 wherein the compound having a boiling point higher than that of methyl borate is selected from among the compounds having a boiling point at least 2°C higher than that of methyl borate.

8. Process according to anyone of claims 1 to 7, wherein the compound having a boiling point higher than that of methyl borate is selected from among hydrocarbons.

9. Process according to anyone of claims 1 to 8, wherein the compound having a boiling point higher than that of methyl borate is selected from the group consisting of cyclohexane and saturated hydrocarbons having 7 to 20 carbon atoms in their molecule.

10. Process according to anyone of claims 1 to 9, wherein water in a quantity of at least 0.5% by weight, based on the weight of the methanol present, is introduced into the decanter.

11. Process according to anyone of claims 1 to 10 wherein the starting product for the preparation of at least one boric oxide, in anhydrous or hydrated form, is methyl borate, the methanol-methyl borate azeotrope or a methanol-methyl borate mixture containing more than 1 mole of methanol per mole of methyl borate.

12. Process according to anyone of claims 1 to 11, wherein the methyl borate used as the starting product for the preparation of at least one boric oxide, in anhydrous or hydrated form, is the product resulting from the reaction of orthoboric acid of formula H₃BO₃ on a methanol excess, based on the theoretical quantity necessary for the formation of methyl borate and optionally in the presence of water.

13. Process for the oxidation of at least one saturated hydrocarbon selected from the group consisting of cyclohexane and cyclic and acyclic saturated hydrocarbons having 7 to 20 carbon atoms in their molecule, into a product incorporating the corresponding alcohol, according to which:
a) said hydrocarbon is oxidized in the liquid phase with the aid of a gas containing molecular oxygen, in the presence of at least one boron compound, forming esters with the alcohol formed during the oxidation and selected from the group consisting of boric oxides, in anhydrous or hydrated form and of general formula
(I) B₂O₃, xH₂O,
in which x is a number between 0 and 3,
b) the reaction mixture obtained in stage a) and which contains at least one ester of said boron compound is hydrolyzed into a product incorporating the orthoboric acid and an alcohol corresponding to said hydrocarbon,
c) the reaction mixture obtained in stage b) is separated into an aqueous phase containing orthoboric acid and an organic phase containing an alcohol corresponding to said hydrocarbon and from which said alcohol is recovered,
said process including the regeneration of boric oxide by a process according to anyone of claims 1 to 12 being characterized in that it comprises the following stages:
d) under methyl borate formation conditions, the aqueous phase obtained in stage c) is treated by methanol in a molar quantity at least equal to the theoretical quantity necessary for the formation of the methyl borate-methanol azeotrope and, in a distillation column, separation takes place of the methyl borate in the form of its azeotrope with methanol and which is recovered at the top of the column, and a phase having a higher boiling point than that of the methanol-methyl borate azeotrope and containing water, which is recovered at the bottom of the column,
e) into a hydrolysis zone are introduced the methanol-methyl borate azeotrope recovered in stage d) and water, in a water:methyl borate molar ratio of 1.3:1 to 2.9:1 and in said zone methyl borate hydrolysis at lease partly takes place,
f) into a distillation column are introduced the product from the hydrolysis zone of stage e), at least one compound forming a heteroazeotrope with methanol, said heteroazeotrope having a boiling point below that of the methanol-methyl borate azeotrope and at least one compound having a boiling point higher than that of the methyl borate, said compound not forming an azeotrope with a boiling point below that of said heteroazeotrope,
g) at the top of the column used in stage f), said heteroazeotrope is recovered and is fed into a decanter, where separation takes place of the methanol from the compound forming said heteroazeotrope and said compound is recycled into the distillation column as reflux and at least partly as a charge of said compound to said column,
h) at the bottom of the column used in stage f), recovery takes place of a mixture incorporating at least one boric oxide, in anhydrous or hydrated form, and said compound with a boiling point higher than that of methyl borate and
i) recycling takes place to stage a) of the mixture recovered in stage h) or the boric oxide obtained by separation from said mixture recovered in stage h).

14. Process according to claim 13, wherein the boron compound is selected from the group consisting of boric anhydride of general formula (I), in which x is equal to zero, metaboric acid of general formula (I), in which x is equal to 1, mixtures of these two compounds and mixtures of metaboric acid and orthoboric acid containing less than 60% by weight orthoboric acid of general formula (I), in which x is equal to 3, in which in stage e) the water-methyl borate molar ratio is approximately 1.5:1 to approximately 2.6:1 and in which the hydrolysis conditions are such that formation takes place of either the boric anhydride, or the metaboric acid, or a mixture of these two compounds, or a mixture incorporating metaboric acid and orthoboric acid, said mixture containing less then 60% by weight orthoboric acid.

15. Process according to claim 13 or 14 wherein stage d) is performed in a reaction-distillation zone.

16. Process according to anyone of claims 13 to 15 wherein the methanol separated in stage g) is recycled to the methyl borate formation stage d).

17. Process according to anyone of claims 13 to 16 wherein the organic phase separated in stage c) undergoes a water-washing prior to the recovery of the alcohol and the washing water is at least partly used in the hydrolysis stage b) of the reaction mixture obtained in stage a).

18. Process according to claim 17, wherein the water used for washing the organic phase separated in stage c) at least partly comes from the methyl borate formation stage d).

19. Process according to anyone of claims 13 to 18 wherein the saturated hydrocarbon is cyclohexane.

20. Process according to anyone of claims 13 to 19 wherein the compound having a higher boiling point than that of the methyl borate used in stage f) is the hydrocarbon subjected to oxidation in stage a).

## Patentansprüche

1. Verfahren zur Herstellung wenigstens eines Boroxides in wasserfreier oder hydratisierter Form mit der allgemeinen Formel
(I) B₂O₃.xH₂O,
worin x eine Zahl von 0 bis 3 ist, durch Hydrolyse von Methylborat, worin man in eine Hydrolysezone Methylborat und Wasser in einem molaren Verhältnis von wenigstens 1,3 Mol Wasser pro Mol Methylborat einführt, man dann das aus der Hydrolysezone austretende Produkt, welches das Boroxid und Methanol enthält und wenigstens eine Verbindung, welche mit Methanol ein Heteroazeotrop bildet, in eine Destillierkolonne einführt, wobei das Heteroazeotrop einen niedrigeren Siedepunkt als das Methyl-/Methanolborat - Azeotrop aufweist, dadurch gekennzeichnet, daß
- man in die Kolonne auch wenigstens eine Verbindung einführt, die einen höheren Siedepunkt als Methylborat aufweist, wobei die Verbindung kein Azeotrop mit niedrigerem Siedepunkt als dem des Heteroazeotrops kein bildet,
- man vom Kolonnenkopf das Heteroazeotrop gewinnt, das man in einen Dekanteur leitet, worin man das Methanol von der das Heteroazeotrop bildenden Verbindung abtrannt und man diese Verbindung in die Destillierkolonne als Rückflußmittel und als Versorgung der Kolonne mit dieser Verbindung zurückführt,
- man am Kolonnenboden eine Mischung aus wenigstens einem Boroxid in wasserfreier oder hydratisierter Form und eine Verbindung mit einem höheren Siedepunkt als dem des Methylborats gewinnt.

2. Verfahren nach Anspruch 1, worin die ein Heteroazeotrop mit Methanol bildende Verbindung aus der Gruppe von Verbindungen ausgewählt ist, welche ein Heteroazeotrop bilden, das wenigstens 10 Gew.% Methanol im Heteroazeotrop enthält.

3. Verfahren nach Anspruch 1 oder 2, worin die ein Heteroazeotrop mit Methanol bildende Verbindung aus jenen ausgewählt ist, deren Heteroazeotrop einen um wenigstens 2°C niedrigeren Siedepunkt als der Siedepunkt des Methanol/Methylborat-Azeotropes hat.

4. Verfahren nach einem der Ansprüche 1 bis 3, worin die ein Heteroazeotrop mit Methanol bildende Verbindung aus den Kohlenwasserstoffen ausgewählt ist.

5. Verfahren nach einem der Ansprüche 1 bis 4, worin die ein Heteroazeotrop mit Methanol bildende Verbindung aus der Gruppe ausgewählt ist, die aus den Kohlenwasserstoffen mit 4 bis 6 Kohlenstoffen im Molekül gebildet ist und keinen Ring mit 6 Kettengliedern enthalten.

6. Verfahren nach einem der Ansprüche 1 bis 5, worin die ein Heteroazeotrop mit Methanol bildende Verbindung aus der Gruppe ausgewählt ist, die aus 2-Methylpentan und 2,3-Dimethylbutan gebildet ist.

7. Verfahren nach einem der Ansprüche 1 bis 6, worin die Verbindung mit höherem Siedepunkt als dem von Methylborat aus den Verbindungen ausgewählt ist, die einen um wenigstens 2°C höheren Siedepunkt als dem von Methylborat aufweisen.

8. Verfahren nach einem der Ansprüche 1 bis 7, worin die Verbindung mit höherem Siedepunkt als dem von Methylborat aus den Kohlenwasserstoffen ausgewählt ist.

9. Verfahren nach einem der Ansprüche 1 bis 8, worin die Verbindung mit höherem Siedepunkt als dem von Methylborat aus der Gruppe ausgewählt ist, die aus Cyclohexan und den gesättigten Kohlenwasserstoffen mit 7 bis 20 Kohlenstoffatomen im Molekül gebildet ist.

10. Verfahren nach einem der Ansprüche 1 bis 9, worin man in den Dekanteur Wasser in einer Menge von wenigstens 0,5 Gew.%, bezogen auf das Gewicht des anwesenden Methanols, einführt.

11. Verfahren nach einem dar Ansprüche 1 bis 10, worin man als Ausgangsprodukt für die Herstellung wenigstens eines Boroxides in wasserfreier oder hydratisierter Form Methylborat, Methanol/Methylborat-Azeotrop oder eine Methanol/Methylborat-Mischung, welche mehr als ein Mol Methanol pro Mol Methylborat enthält, verwendet.

12. Verfahren nach einem der Ansprüche 1 bis 11, worin das als Ausgangsprodukt für die Herstellung wenigstens eines Boroxides in wasserfreier oder hydratisierter Form verwendete Methylborat das Produkt ist, das aus der Umsetzung von Orthoborsäure der Formel H₃BO₃ über einem Überschuß von Methanol, bezogen auf die zur Bildung für Methylborat theoretisch erforderliche Menge, gegebenenfalls in Gegenwart von Wasser, resultiert.

13. Verfahren der Oxidation wenigstens eines gesättigten Kohlenwasserstoffs, der aus der Gruppe ausgewählt ist, die aus Cyclohexan und den acyclischen und cyclischen Kohlenwasserstoffen mit 7 bis 20 Kohlenstoffen im Molekül gebildet ist, in ein Produkt, das den entsprechenden Alkohol enthält, wonach:
a) man den Kohlenwasserstoff in flüssiger Phase mit Hilfe eines molekularen Sauerstoff enthaltenden Gases in Gegenwart wenigstens einer Borverbindung oxidiert, welche mit dem während der Oxidation gebildeten Alkohol Ester bildet und aus der Gruppe ausgewählt ist, die aus Boroxiden in wasserfreier oder hydratisierter Form der allgemeinen Formel
(I) B₂O₃.x H₂O,
worin x eine Zahl von 0 bis 3 ist, gebildet ist,
b) man die in Schritt a) erhaltene Reaktionsmischung, welche wenigstens einen Ester der Borverbindung enthält, in ein Produkt hydrolysiert, das Orthoborsäure und einen dem Kohlenwasserstoff entsprechenden Alkohol umfaßt,
c) man die in Schritt b) erhaltene Reaktionsmischung in eine wäßrige Phase, die Orthoborsäure enthält, und eine organische Phase, die einen dem Kohlenwasserstoff entsprechenden Alkohol enthält, aus welcher man den Alkohol gewinnt, auftrennt,
wobei das Vorfahren die Regenerierung von Boroxiden durch ein Verfahren nach einem der Ansprüche 1 bis 12 beinhaltet und dadurch gekennzeichnet ist, daß es die folgende Schritte umfaßt:
d) man behandelt unter Bedingungen der Methylboratbildung die in Schritt c) erhaltene wäßrige Phase mit Methanol, in einer molaren Menge, die wenigstens gleich der für die Bildung des Methyl-/Methanol-Azeotrops notwendigen theoretischen Menge ist und man trennt in einer Destillierkolonne das Methylborat in Form seines Azeotropes mit Methanol, das Azeotrop, das man am Kolonnenkopf gewinnt und eine Phase mit viel höherem Siedepunkt als jenem des Methanol/Methylborat-Azeotropes, das Wasser enthält, das man am Kolonnenboden gewinnt, auf,
e) man führt in eine Hydrolysezone das in Schritt d) gewonnene Methanol/Methylborat-Azeotrop und Wasser in einem molaren Verhältnis von Wasser/Methylborat von 1,3:1 bis 2,9: 1 ein und man bewirkt in dieser Zone wenigstens einen Teil der Methylborathydrolyse,
f) man führt in eine Destillierkolonne ein:
- das aus der Hydrolysezone von Schritt e) stammende Produkt,
- wenigstens eine Verbindung, die mit dem Methanol ein Heteroazeotrop bildet, wobei das Heteroazeotrop einen Siedepunkt aufweist, der niedriger ist als der des Methanol/Methylborat-Azeotrops und
- wenigstens eine Verbindung mit einem Siedepunkt, der höher ist als der des Methylborats, wobei die Verbindung kein Azeotrop mit niedrigerem Siedepunkt als dem des Heteroazeotrops bildet,
g) man gewinnt am in Schritt f) verwendeten Kolonnenkopf das Heteroazeotrop, das man in einen Dekanteut einführt, worin man das Methanol von der Verbindung, die das Heteroazeotrop bildet abtrennt und man diese Verbindung in die Destillierkolonne als Rückflußmittel und wenigstens teilweise als Versorgung der Kolonne mit dieser Verbindung zurückführt,
h) man gewinnt am in Schritt f) verwendeten Kolonnenboden eine Mischung, welche wenigstens ein Boroxid in wasserfreier oder hydratisierter Form und die Verbindung mit höherem Siedepunkt als dem des Methylborats umfaßt und
i) man führt die im Schritt h) gewonnene Mischung oder das durch Abtrennung aus der in Schritt h) gewonnenen Mischung erhaltene Boroxid in Schritt a) zurück.

14. Verfahren nach Anspruch 13, worin die Borverbindung aus der Gruppe ausgewählt ist, die aus Borsäureanhydrid der allgemeinen Formel (I), worin x gleich Null ist, Metaborsäure der allgemeinen Formel (I), worin x gleich 1 ist, den Mischungen dieser beiden Verbindungen und den Mischungen von Metaborsäure und Orthoborsäure, welche wenigstens 60 Gew.% Orthoborsäure der allgemeinen Formel (I), worin x gleich 3 ist, gebildet ist, worin in Schritt e) das Molverhältnis Wasser/Methylborat etwa 1,5:1 bis etwa 2,6:1 beträgt und worin die Hydrolysebedingungen derart sind, daß man entweder Borsäureanhydrid, Metaborsäure , eine Mischung dieser beiden Verbindungen oder eine Mischung bildet, die Metaborsäure und Orthoborsäure umfaßt, wobei die Mischung wenigstens 60 Gew.% Orthoborsäure enthält.

15. Verfahren nach Anspruch 13 oder 14, worin der Schritt d) in einer Reaktions-/Destillationszone bewirkt wird.

16. Verfahren nach einem der Ansprüche 13 bis 15, worin das in Schritt g) abgetrennte Methnol in Schritt d) der Bildung von Methylborat zurückgeführt wird.

17. Verfahren nach einem der Ansprüche 13 bis 16, worin die in Schritt c) abgetrennte organische Phase vor der Gewinnung von Alkohol einem waschen mit Wasser unterworfen wird und das Waschwasser wenigstens teilweise im Schritt b) der Hydrolyse der in Schritt a) erhaltenen Reaktionsmischung verwendet wird.

18. Verfahren nach Anspruch 17, worin das zum Waschen der in Schritt c) abgetrennten organischen Phase verwendete Wasser wenigstens teilweise aus Schritt d) der Methylboratbildung stammt.

19. Verfahren nach einem der Ansprüche 13 bis 18, worin der gesättigte Kohlenwasserstoff Cyclohexan ist.

20. Verfahren nach einem der Ansprüche 13 bis 19, worin die in Schritt f) verwendete Verbindung mit höherem Siedepunkt als dem von Methylborat Kohlenwasserstoff ist, der in Schritt a) der Oxidation unterworfen wird.
